# EUROPÄISCHE PATENTSCHRIFT

(11) **EP 0 260 610 B1**
(45) Veröffentlichungstag und Bekanntmachung des Hinweises auf die Patenterteilung: **01.09.1993**
(21) Anmeldenummer: 87113278.3
(22) Anmeldetag: 11.09.1987
(51) Int. Cl.: C12P 21/00, C12N 15/00, G01N 33/577, A61K 39/395

(54) **Monoklonale Antikörper gegen humanen Tumonekrosefaktor (TNF) und deren Verwendung**
Monoclonal antibodies against human tumour necrotic factor (TNF), and their use
Anticorps monoclonaux contre le facteur nécrotique tumoral humain (TNF) et leur utilisation

(30) Priorität: 13.09.1986 DE 3631229
(43) Veröffentlichungstag der Anmeldung: 23.03.1988
(73) Patentinhaber: BASF Aktiengesellschaft, 67063 Ludwigshafen (DE)
(72) Erfinder: Moeller, Achim, Dr., D-6703 Limburgerhof (DE); Emling, Franz, Dr., D-6700 Ludwigshafen (DE)

(56) Entgegenhaltungen:
- EP-A- 0 218 868
- CHEMICAL ABSTRACTS, Band 105, Nr. 25, 22. Dezember 1986, Seite 612, Zusammenfassung Nr. 224436w, Columbus, Ohio, US
- CHEMICAL ABSTRACTS, Band 105, Nr. 23, 8. Dezember 1986, Seite 451, Zusammenfassung Nr. 207220d, Columbus, Ohio, US; H. HAYASHI et al.: "An enzyme-linked immunosorbent assay for recombinant human tumor necrosis factor using monoclonal antibody"
- CHEMICAL ABSTRACTS, Band 105, Nr. 5, 4. August 1986, Seite 579, Zusammenfassung Nr. 40912s, Columbus, Ohio, US; C.M. LIANG et al.: "Production and characterization of monoclonal antibodies against recombinant human tumor necrosis factor/cachectin"
- CHEMICAL ABSTRACTS, Band 104, Nr. 11, 17. März 1986, Seite 534, Zusammenfassung Nr. 86989e, Columbus, Ohio, US
- CHEMICAL ABSTRACTS, Band 104, Nr. 9, 3. März 1986, Seite 562, Zusammenfassung Nr. 67346b, Columbus, Ohio, US

## Beschreibung

Die Erfindung betrifft eine Hybridomzellinie, die einen hochspezifischen monoklonalen Antikörper (mAK) gegen TNF synthetisiert, diesen monoklonalen Antikörper gegen TNF, Verfahren zur Herstellung dieser Hybridomzellinie und dieses Antikörpers sowie die Verwendung des monoklonalen Antikörpers.

Die Fusion von Maus-Myelomzellen mit Milzzellen von immunisierten Mäusen (Köhler und Milstein, Nature 256, 495 - 497 (1975)) war der erste Hinweis für die Möglichkeit, kontinuierliche Zellinien zu erhalten, die einheitliche (sog. "monoklonale") Antikörper produzieren. Seitdem sind zahlreiche Versuche unternommen worden, verschiedene Hybridzellen (sogenannte: Hybridome) herzustellen und die Antikörper, die von ihnen gebildet werden, für unterschiedliche wissenschaftliche Untersuchungen einzusetzen (vgl. Current Topics in Microbiology and Immunology Vol. 81: Lymphocyte Hybridomas, Springer Verlag 1978).

Durch seine biologischen Eigenschaften scheint sich TNF als interessantes und vielversprechendes Mittel bei der Behandlung von Tumorerkrankungen einsetzen zu lassen. Eingehende Untersuchungen scheiterten zunächst an der äußerst geringen Konzentration an TNF in natürlichen Zellen. Erst mit der Entwicklung der Gentechnologie und der Möglichkeit, humanes Protein in niederen Organismen klonieren zu können, war es möglich, auch TNF in Mikroorganismen zu exprimieren. Dieser rekombinante TNF (rTNF) zeigt in hochgereinigter Form die gleichen Wirkungen wie natürlicher TNF (nTNF).

Wissenschaftliche Untersuchungen, ebenso wie die therapeutische Anwendung, lassen das Bedürfnis entstehen, nicht nur die Aktivität des TNF, sondern das Protein TNF selbst, nachzuweisen. Die Bestimmung der biologischen Aktivität ist nur mühsam und aufwendig durchzuführen.

Es ist hingegen bekannt, daß man mit Antikörpern gegen TNF die Wirkung von TNF blockieren kann (Biochem. Biophys. Res. Comn. 137, 847 (1986)). In der genannten Publikation wird über solche Antikörper berichtet, die eine relativ hohe Affinität zu TNF besitzen sollen. Es ist darin jedoch kein nacharbeitbarer Weg zu deren Herstellung angegeben worden.

Gegenstand der vorliegenden Erfindung ist ein neuer monoklonaler Maus-Antikörper gegen humanen natürlichen und rekombinanten TNF, die sie produzierende Hybridzellinie, Verfahren zu seiner Herstellung und seine Verwendung.

Die Herstellung des monoklonalen Antikörpers erfolgte in Anlehnung an bekannte Methoden (Monoclonal Antibodies, Kennet et al., Plenum Press 1980, S. 363 - 419).

Durch wiederholte Injektion einer geringen Menge des gereinigten, rekombinanten TNF's aus E.coli wurden BALB/c-Mäuse immunisiert. Sobald im Serum genügend Antikörper nachweisbar waren, wurden die Milzzellen dieser Tiere mit Myelomzellen fusioniert und die Hybride kultiviert.

Die einzelnen Kulturen wurden auf ihren Gehalt an spezifischen Antikörpern gegen TNF mit Hilfe eines Screening-Tests untersucht.

Von geeigneten Hybridomen wurde nach der Methode des "limiting dilution cloning" Kolonien isoliert, die sich von Einzelzellen ableiteten. Auf diese Weise erhielt man unter anderem eine Hybridzellinie, die sich durch die Sekretion des monoklonalen Antikörpers mAK 195 auszeichnet. Diese Zellinie ist bei der European Collection of Animal Cell Cultures (ECACC), PHLS Centre for Applied Microbiology and Research, Porton Down, Salisbury SP4 OJ6 in Großbritannien unter der Nummer 87 050801 hinterlegt. Zwei weitere Zellinien (AM-1 und AM-199) sind unter den Nummern 87 050804 und 87 050802 hinterlegt.

Zur Vermehrung dieser Hybridzellen wurden sie sowohl in vitro als auch in vivo kultiviert. Die hohe Vermehrungsrate in vivo machte dieses Kulturverfahren besonders günstig. Dazu wurden mit Pristan® vorbehandelten BALB/c-Mäusen Zellen der einzelnen Hybridstämme intraperitoneal injiziert. Der sich bildende Ascitestumor wurde nach ca. 8 bis 10 Tagen abgeerntet.

Die Isolierung der monoklonalen Antikörper gegen TNF erfolgte durch die Aufreinigung von entweder den Überständen der in vitro Zellkultur oder der Ascitesflüssigkeit. Die Reinigung erfolgte in Anlehnung an die Methode von Bruch et al. (J. Immunol. Methods 1982, Vol. 53, 313 - 319).

Die Charakterisierung des molekularen Eigenschaften der Antikörpers mAK 195 ergab:
- Das Molekulargewicht des gereinigten Antikörper beträgt größer/gleich 150 000 Dalton (Bestimmung durch Polyacrylamidgelelektrophorese).
- Der Antikörper mAK-195 ist vom Typ IgG3, wobei seine schwere Kette gamma 3 ist. Die leichte Kette ist kappa (Bestimmung durch subtypenspezifische Antikörper in einem ELISA).

Der monoklonale Antikörper zeichnet sich durch eine hohe Affinitätskonstante in der Größenordnung >10⁹ l/mol gegen TNF aus und zeigt keine Kreuzreaktion mit Lymphotoxin.

Die relative Lage der Bindungsstellen für Antikörper auf dem TNF Molekül wurde durch kompetitive Bindung des Antikörpers untersucht.

Die Aktivität von TNF wurde in einem üblichen cytotoxischen Test bestimmt. Rekombinanter und nativer TNF wurden mit einem Überschuß an Antikörpern inkubiert. Die cytotoxische Aktivität beider TNF-Präparate wurde durch den Antikörper mAK-195 neutralisiert. Das bedeutet, daß der mAK-195 mit einer für die biologische Aktivität verantwortlichen Region reagiert.

Sollen Antikörper zum Nachweis eines bestimmten Antigens dienen, sind im Prinzip zwei Testanordnungen möglich. In beiden muß, wenn es keinen natürlichen Marker im Antigen gibt, eine Markierung einer der Komponenten vorgenommen werden. Entweder erfolgt diese am Antigen, z. B. durch ein radioaktives Marker-Isotop, dann wird meist ein kompetitiver Verdrängungsassay benutzt wie der bekannte Radio-Immuno-Assay (RIA) oder die Markierung erfolgt am Antikörper, wobei der bevorzugte Testtyp ein Immuno-Radiometrischer-Assay (IRMA) ein Enzyme-Linked-Immunosorbant-Assay (ELISA) oder ein Chemilumineszenz-Assay ist. Einzelheiten dieser verschiedenen Testmethoden und Verfahrensvarianten sind dem Fachmann bekannt.

Eine andere Möglichkeit besteht in der Kopplung mit niedermolekularen Haptenen an Antikörper, die ihrerseits durch eine zweite Reaktion spezifisch nachgewiesen werden können. Gebräuchlich sind z. B. Biotin reagierend mit Streptavidin. Der erfindungsgemäße Antikörper wurde daher mit long chain-Biotin markiert und in einem Nachfolgeschritt mit Streptavidin-Meerrettichperoxidase-Komplex sichtbar gemacht.

Der hier beschriebene Test stellt einen Festphasen sandwich-ELISA dar. Ein nicht markierter Antikörper (mAK-1 oder mAK-199) wurde an eine Oberfläche, z. B. Mikrotiterplatten, passiv adsorbiert oder kovalent gebunden und die Oberfläche in bekannter Weise gegen unspezifische Bindung blockiert. TNF-haltige Proben und der mit Biotin-markierte Antikörper mAK-195 wurden in die Näpfchen pipettiert und inkubiert. Es konnte gezeigt werden, daß TNF mit einer Nachweisgrenze von 10 pg/ml in Proben nachzuweisen ist. rTNF hat eine spezifische Aktivität von 8,0 x 10⁶ U/mg im Maus L929 Test, so daß mit diesem ELISA 0,1 U TNF/ml nachgewiesen werden können. Durch Western-Blotting konnte gezeigt werden, daß der Antikörper mit keiner Komponente im menschlichen Serum kreuzreagiert. Der erfindungsgemäße Antikörper könnte daher zur Bestimmung von TNF im Serum von mit TNF behandelten Patienten verwendet werden. Er ist ferner für aktuelle diagnostische Zwecke, z. B. zur Überprüfung des TNF-Spiegels im Serum und Plasma, einsetzbar.

Da der erfindungsgemäßen Antikörper TNF inaktiviert (vgl. Beispiel 6), kann er zur Behandlung von Krankheiten eingesetzt werden, bei denen die Konzentration an TNF im Blut erhöht ist, wie z.B. bei septischem Schock. Weiter kann bei folgenden Erkrankungen eine Behandlung mit den TNF-Antikörper angezeigt sein; Transplantatabstoßung, Allergien, Antoimmunkrankheiten, Erkrankungen des rheumatischen Formenkreises, Schocklunge, entzündliche Knochenerkrankungen, Blutgerinnungsstörungen, Verbrennungen.

Mittels Immunaffinitätschromatographie kann auch TNF aus biologischem Material, das TNF enthält, aufgereinigt werden. Hierzu wird der Antikörper nach bekannten Methoden an eine Gelmatrix gebunden, über die die TNF-haltige Lösung gegeben wird.

Die nachfolgenden Beispiele sollen die Erfindung näher erläutern:

### Beispiel 1:

### Herstellung von monoklonalen Antikörpern

a) Immunisierung von BALB/c-Mäusen
Weibliche BALB/c-Mäuse wurden intraperitoneal (i. p.) mit 30 µg rTNF in 0,5 ml komplettem Freund Adjuvant immunisiert. 14 Tage später erhielten die Tiere erneut 30 µg des Antigens intraperitoneal in inkomplettem Freund Adjuvant. Zwei weitere Immunisierungen erfolgten intraperitoneal in 14tägigen Abständen mit jeweils 30 µg Antigen. Drei Tage nach der letzten Antigengabe wurden die Milzen von 2 Tieren entnommen.
b) Präparation einer Milzzellsuspension
Aus den entnommenen Milzen wurde eine Zellsuspensionen hergestellt, in dem die Organe durch ein Sieb aus rostfreiem Stahl (Porenweite 100 µm) gepreßt wurden. Die Zellen wurden in Dulbecco Minimal Essential Medium (DMEM), das mit 4,5 g/l Glucose, 10 mM Glutamin, 1000 Einheiten/ml Penicilin, 100 µg/ml Streptomycin und 15 % foetales Kälberserum supplementiert war, überführt. Die Zellen wurden dreimal mit Medium gewaschen und dann in gewünschter Konzentration in demselben Medium resuspendiert. Im allgemeinen wurden etwa 5 bis 10 x 10⁷ Zellen pro Milz gewonnen.
c) Wachstum der Myelomzellen
Als Fusionspartner wurden die Myelomzellen Sp2/0-Ag14 (ATCC No. CRL 8287) verwendet. Die Zellen sind gegen 20 µg/ml 8-Azaguanin resistent und können im Medium, das Hypoxanthin, Aminopterin und Thymidin (HAT) enhält, nicht mehr wachsen. Sie wurden in DMEM, das mit 4,5 g/l Glucose, 10 mM Glutamin, 1000 Einheiten/ml Penicilin, 100 µg/ml Streptomycin und 15 % foetales Kälberserum supplementiert war, kultiviert (komplettes Medium). Zur Fusion wurden die Zellen in der logarithmischen Wachstumsphase verwendet.
d) Zellfusion
Die Milzzellsuspensionen wurden mit den Myelomzellen im Verhältnis 5 : 1 gemischt und in serumfreien DMEM gewaschen. Die gewaschenen Zellen wurden in 30 ml serumfreien DMEM resuspendiert und in einem 50 ml konischen Polypropylen-Röhrchen bei 800 U/min 5 Minuten zentrifugiert, Der Überstand wurde vollständig abgesaugt. Auf das Pellet wurde ganz vorsichtig 0,5 ml einer 50 %igen Polyethylenglykollösung (PEG, Boehringer) vom Molekulargewicht 2000 gegeben, das Zellpellet durch leichtes Klopfen mit PEG vermischt, und anschließend bei 1000 U/min drei Minuten zentrifugiert. Daraufhin wurden 10 ml DMEM zugegeben, das Zellpellet vorsichtig gelöst und anschließend drei Minuten bei 2000 U/min abzentrifugiert. Das Zellpellet wurde in HAT-Medium in einer Konzentration von 2 x 10⁶ Zellen/ml resuspendiert und in 0,2 ml Anteilen auf Mikrotiterplatten verteilt. In die Näpfchen waren am Tag zuvor etwa 50000 peritoneale mononucleare Zellen, vornehmlich Makrophagen als Fütterzellen, gegeben worden.
e) Selektionierung und Anzucht der Hybridome
Nach der Zellfusion wurden die Zellen im HAT-Medium nach Littlefield (Science 1964 Vol. 145, 709 - 712) bei 37°C mit 5 % CO₂ in einer feuchten Atmosphäre kultiviert. Die Kulturen werden zweimal in der Woche durch Ersetzen des halben Mediums durch frisches HAT Medium gefüttert. Nach einigen Wochen wurden Überstände von Hybridomzellkulturen auf das Vorhandensein von Anti-human-Tumornekrosefaktoraktivität untersucht. Diejenigen Hybridome, die positive Resultate im Screening-Test zeigten, wurden zum Klonieren ausgewählt. Dabei wurden die Hybridome einer "Limiting-Dilution"-Technik unterworfen, bei der in 96 Mikrotitervertiefungen im Mittel 0,5 Zellen/Näpfchen ausgesät wurden und 10⁵ Mäusethymocyten als "Feeder-Zellen" zugegeben wurden. Die nach diesem Klonierungsverfahren selektionierten antikörperproduzierenden Zellen wurden vermehrt, eingefroren und in flüssigem Stickstoff in komplettem Medium, das 10 % foetales Kälberserum sowie 10 % Dimethylsulfoxid enthielt, aufbewahrt.
f) Screening-Test auf spezifische TNF-Antikörper
rTNF wurde in PBS (Phosphat gepufferte Salzlösung, bestehend aus 0,8 % NaCl und 0,02 molar Natriumphosphat, eingestellt mit HCl oder NaOH auf pH 7,4) auf 3 µg/ml verdünnt. Näpfchen von Mikrotiterplatten® wurden mit je 0,1 ml dieser Lösung beschickt. Nach zwei Stunden bei Raumtemperatur wurde der Überstand abgesaugt und die Näpfchen mit 0,3 ml einer 1 %igen Rinderserumalbumin-Lösung (Sigma, RIA grade) mindestens 30 Minuten behandelt. Danach wurde der Überstand verworfen. Überstände von wachsenden Hybridomzellinien, die ungefähr 20 - 30 % konfluent waren, oder Serumverdünnungen von immunisierten Mäusen wurden für mindestens zwei Stunden bei Raumtemperatur inkubiert. Die Näpfchen wurden mit 0,3 ml PBS mehrfach gewaschen. Dann wurde mit 0,1 ml einer geeigneten Konzentration an Anti-Maus-Immunglobulin Antikörpern (Miles) für zwei Stunden bei Raumtemperatur inkubiert. Diese Antikörper waren mit dem Enzymmarker Peroxidase gekoppelt. Näpfchen mit positiver Peroxidasereaktion zeigten Antigenspezifische Antikörper an.
Von 360 angelegten Urnäpfchen zeigten 80 % Zellbewuchs. Von diesen zeigten 12 positive Ergebnisse im TNF-Screening-Test. Von diesen wurden 11 wiederholt positiv gewertet. Drei verschiedene monoklonale Hybridome wurden für die vorliegende Erfindung weiterverfolgt: AM-1, AM-195, AM-199.
g) Expansion von Hybridoma-Zellkulturen
Expansion in der Zellkultur (in vitro):
Etwa 2 x 10⁷ Zellen wurden in Zellkulturflaschen mit 175 cm² Wachstumsfläche ausgesät. Der zellfreie Überstand enthielt nach drei Tagen die von den Zellen sekretierten monoklonalen Antikörper im Bereich von 10 bis 20 µg/ml.
Expansion in der Ascites-Maus (in vivo):
BALB/c-Mäuse erhielten zur Konditionierung des Peritoneums 0,5 ml i. p. Pristan®. Innerhalb eines Zeitraumes von 1 bis 2 Wochen wurde den vorbehandelten Tieren eine Suspension von 5 bis 10 x 10⁶ Hybridoma in PBS pro Tier i, p. appliziert. Nach 8 bis 10 Tagen wurde mit einer Kanüle das Peritoneum angestochen und die zellhaltige Ascitesflüssigkeit gesammelt.
Von der entnommenen Ascitesflüssigkeit wurden die zellulären Bestandteile durch Zentrifugation abgetrennt (5000 rpm, 5 Minuten). Der Überstand, der die monoklonalen Antikörper enthielt, wurde in Aliquots bei -70°C eingefroren oder chromatographisch bis zu mindestens 90 % gereinigt, wie durch Natriumdodecylsulfat-polyacrylamid Gelelektrophorese beurteilt wurde (DE-OS 3330160).
h) Typisierung der monoklonalen Antikörper
Die monoklonalen Antikörper wurden in einem ELISA-System näher charakterisiert. Die Vertiefungen einer Mikrotiterplatte wurden mit rTNF (5 µg/ml) beschichtet. Nach Inkubation für 2 Stunden bei Raumtemperatur dieser so vorbereiteten Platte mit aufgereinigten monoklonalen Antikörpern aus Zellkultur folgte ein zweiter Inkubationsschritt (2 Stunden bei Raumtemperatur) mit Anti-Maus-Immunglobulin verschiedener Klassen und Subklassen sowie mit verschiedenen Klassen leichter und schwerer Immunglobulinketten (Miles). In einem weiteren Schritt wurde ein Peroxidase-markiertes Ziegen-Anti-Kaninchen (Miles) Immunglobulin zugesetzt. Nach Inkubation von einer Stunde wurde die Enzymreaktion durch Zugabe des Farbsubstrates Tetramethylbenzidine (Miles) und Wasserstoffperoxid gestartet. Die Ergebnisse sind in Tabelle 1 zusammengefaßt.

**Tabelle 1**

| Bezeichnung des Antikörpers | Klasse | Kette | |
|---|---|---|---|
| | | schwere | leichte |
| mAK-1 | IgG | gamma 1 | kappa |
| mAK-195 | IgG | gamma 3 | kappa |
| mAK-199 | IgG | gamma 1 | kappa |

i) Markierung der monoklonalen Antikörper
NHS-LC-Biotin (Pierce) wurde in PBS gelöst und auf eine Konzentration von 1 mg/ml eingestellt. 0,1 ml dieser Lösung wurden mit 0,1 ml von aufgereinigten monoklonalen Antikörpern (0,5 mg/ml PBS) gemischt und die Lösung für zwei Stunden bei Raumtemperatur inkubiert. Im Anschluß an die Reaktion wurde die Lösung auf 1 ml mit PBS aufgefüllt und bei 4°C gegen PBS dialysiert. Das Dialysat, das heißt, die im Dialyserohr befindliche Lösung, wurde bis zum Gebrauch bei 4°C aufbewahrt.

### Beispiel 2

### Affinität der monoklonalen Antikörper zu TNF

Die Affinitätskonstanten wurden anhand von Daten aus einem ELISA bestimmt. Hierbei wurden die gereinigten Antikörper gegen eine konstante Menge TNF titriert und die Bindung der Antikörper durch Bindung von Peroxidase-markierten Kaninchen Anti-Maus Immunglobulin nachgewiesen. Die Bindungsdaten wurden mit einem speziellen Programm ausgewertet.

**Tabelle 2**

| Affinitätskonstanten der monoklonalen Antikörper | |
|---|---|
| Bezeichnung des Antikörpers | Kₐ x 10⁹ 1 x Mol⁻¹ |
| mAK-1 | 1,5 ± 30 % |
| mAK-195 | 3,5 ± 30 % |
| mAK-199 | 2,0 ± 30 % |

Die gezeigte Assoziationskonstante Kₐ weist den anti-TNF Antikörper mAK-195 als hochaffinen Antikörper aus.

### Beispiel 3

### Neutralisation der cytotoxischen Aktivität von TNF

Die biologische Aktivität von TNF in vitro wurde, wie bei Aggarwal et al. (J. Biol. Chem. 1985, Vol. 260, 2345 - 2354) beschrieben, durch Lyse der Mauszellinie L929 (ATCC Nr. CCL1) bestimmt. Bei Versuchen zur Neutralisation der cytotoxischen Aktivität von TNF durch den monoklonalen Antikörper, wurde eine Konzentration an TNF gewählt, bei der mindestens 90 % der Zellen lysierten. Der Antikörper wurden in komplettem Medium in 1 : 2 Schritten in Mikrotiterplatten verdünnt. Zu jeder Antikörperlösung (0,1 ml) wurden 0,05 ml rekombinanter oder nativer TNF (1,3 ng/ml) gegeben und zwei Stunden bei Raumtemperatur inkubiert. Anschließend erfolgte Zugabe von 50 000 L929 Zellen in 0,05 ml Medium und nach einer Inkubation von 20 bis 24 Stunden im Brutschrank wurden die Zellen fixiert und mit Kristallviolett gefärbt.

Der cytotoxische Effekt von TNF läßt die Zellen lysieren und damit während der Färbung abschwemmen. Ist jedoch genügend Antikörper vorhanden, so wird der cytotoxische Effekt von TNF neutralisiert und die Zellen werden gefärbt.

### Beispiel 4

### Bestimmung von TNF

a) Auswahl eines geeigneten Paares von monoklonalen Antikörpern
   Zur Unterscheidung zwischen kompetitiver Bindung von zwei Antikörpern an dasselbe Epitop und additiver Bindung an verschiedene Epitope wurde die Bindung der Antikörper allein und in Paaren in allen möglichen Kombinationen an immobilisiertem TNF getestet. TNF wurde, wie in Beispiel 1 beschrieben, gebunden. Aufgereinigte Antikörper, beginnend mit 24000ng/ml, wurden in Schritten von 1 : 4 verdünnt. Anschließend folgte die Zugabe von biotinyliertem Antikörper mAK-195 und Inkubation für 90 Minuten. Die Näpfchen wurden mit PBS 0,05 % Tween® 20 gewaschen, Streptavidin-Peroxidase-Komplex (BRL) hinzugegeben und für 30 Minuten inkubiert. Nach einem Waschschritt wurde 0,1 ml Peroxidase-Substrat (siehe Beispiel 4 b) pro Näpfchen hinzugegeben. Bei einer kompetitiven Bindung kommt es zu einer Auslöschung des Signals. Wie aus Tabelle 4 zu entnehmen ist, bindet der monoklonale Antikörper AM-195 an ein anderes Epitop von TNF wie mAK-1 oder mAK-199.

**Tabelle 4**

| Einfluß der Bindung von Biotin-markiertem mAK-195 an TNF durch mAK | | | |
|---|---|---|---|
| µm mAK/ml | 195 | % Bindung | |
| | | 199 | 1 |
| 24000 | 3 | 100 | 100 |
| 6000 | 4 | 100 | 100 |
| 1500 | 16 | 100 | 100 |
| 375 | 63 | 100 | 100 |
| 62 | 95 | 100 | 100 |
| 0 | 100 | 100 | 100 |

b) Aufbau eines Enzymimmun-Tests
   Der Antikörper mAK-1 oder mAK-199 wurde durch passive Adsorption oder kovalente Bindung an einen Träger (Kügelchen, Filter, Mikrotiterplatte aus Polystyrol oder Polyvinylchlorid, Filterpapier oder anderen Materialien) immobilisiert. Die Spezifität von monoklonalen Antikörpern erlaubt nur dem spezifischen Antigen, hier dem TNF, durch Immunbindung, über eine einzige molekulare Bindungsstelle des Antigens gebunden zu werden. Die Menge des bindbaren TNF's ist der Konzentration und Menge des Antigen in der Lösung proportional. Das Antigen wird durch Immunbindung des Antikörpers mAK-195 an eine andere molekulare Bindungsstelle erkannt. Der Antikörper mAK-195 trägt ein Signal. Die Menge des immobilsierbaren Signals ist damit direkt proportional der Menge des immobilisierten Antigens und damit auch der Konzentration des Antigens in der zu untersuchenden Lösung.

### Durchführung des Tests

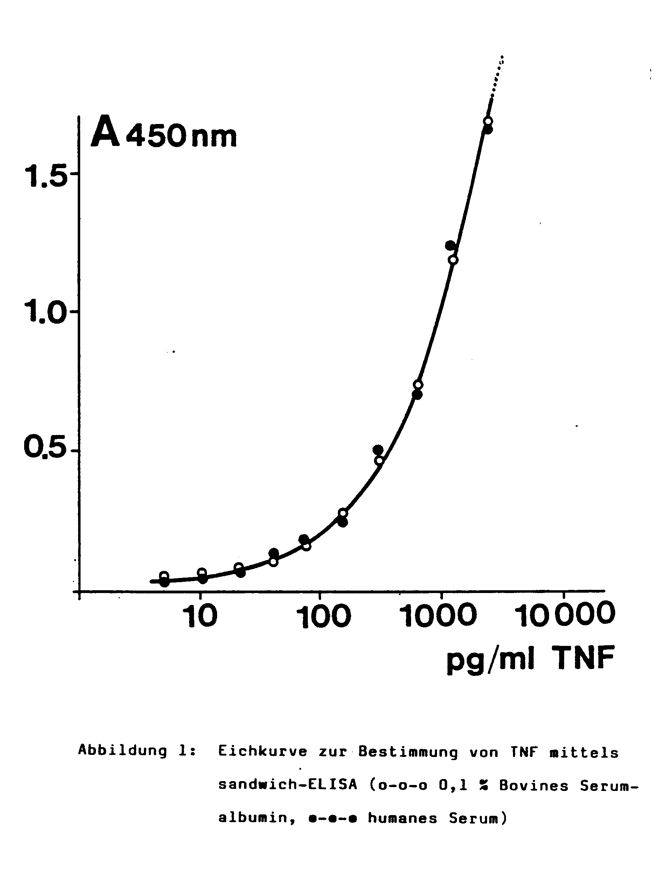

1. Beschichtung
   Aufgereinigte Antikörper mAK-1 oder mAK-199 wurden in Anhaftpuffer (Natrium-Bicarbonatpuffer pH 9,5, 4,2 g/l = 0,05 M) auf 5 µg/ml verdünnt. Die Näpfchen einer Mikrotiterplatte wurden mit 0,1 ml dieser Lösung für 16 bis 20 Stunden bei 4°C inkubiert.
2. Blockierung
   Die gemäß 1 erhaltene Lösung wurde abgesaugt und 2mal mit PBS (2 g/1 NaCl, 0,2 g/l KCl, 1,44 g/l Na₂HPO₄ x 2H2O, 0,2 g/l KH₂PO₄, pH 7,0) gewaschen. Anschließend wurde für 30 Minuten bei Raumtemperatur mit 1 %iger Rinderserumalbumin-Lösung (Sigma, RIA grade) blockiert.
3. Verdünnungsreihe und TNF-Proben
   Die Lösung gemäß 2 wurde abgesaugt und 2mal mit PBS gewaschen. rTNF wurde mit Puffer I (zu 1 l PBS werden 1 g Rinderserumalbumin gegeben, Sigma RIA grade) auf 2,5 ng/ml eingestellt und in 1 : 2 Schritten verdünnt. Je 0,1 ml wurden pro Näpfchen pipettiert und 2-4 Stunden bei Raumtemperatur inkubiert. Nach 3maligem Waschen mit Puffer (Waschpuffer: PBS + 0,1 % Tween® 20) wurden 0,1 ml Biotinmarkierter Antikörper mAK-195 hinzugegeben. Das Konjugat, nach der Vorschrift im Beispiel 1 h) hergestellt, wurde 1 : 400 mit Puffer I verdünnt und für 2 Stunden bei Raumtemperatur oder 16-20 Stunden bei 4-10°C inkubiert.
4. Verstärkersystem
   Die Näpfchen wurden mit Waschpuffer 3mal gewaschen und anschließend mit 0,1 ml Streptavidin-Peroxidase-Komplex (BRL, verdünnt 1 : 2000 in PBS/BSA-Puffer) für 30 min bei Raumtemperatur inkubiert.
5. Entwicklung
   Die Näpfchen wurden 3mal mit Waschpuffer gewaschen, 0,1 ml/Näpfchen Peroxidasesubstrat pipettiert und 30 Minuten bei Raumtemperatur inkubiert. Die Reaktion wurde mit 0,1 ml/Näpfchen 2M H₂SO₄ abgestoppt. Die Ablesung der Absorption in der Mikrotiterplatte erfolgte innerhalb einer Stunde bei einer Wellenlänge von 450 nm. Eine charakteristische Eichkurve ist in Abb. 1 dargestellt. Die Nachweisgrenze für TNF liegt bei 10 pg/ml.

### Peroxidasesubstrat

- TMB-Lösung: 42 mM TMB (3,3',5,5'-Tetramethylbenzidin, Miles) in DMSO
- Substratpuffer: 50 g Natriumacetat auf 1 l Wasser geben und mit 1 g Zitronensäure auf pH 4,9 einstellen.
- 0,1 ml der TMB-Lösung wird langsam und unter Schütteln zu 10 ml Substratpuffer gegeben, gefolgt von 1,47 µl von 30 % H₂O₂ (reinst, Merck).

c) Bestimmung von TNF in Humanserum
   Rekombinantes und/oder natives TNF wurde in Puffer I (Beispiel 4 b) oder in Humanserum auf 2,5 ng/ml eingestellt und in 1 : 2 Schritten unter den gleichen Bedingungen verdünnt. Je 0,1 ml wurden pro Näpfchen pipettiert, die vorher, wie unter Beispiel 4 b) beschrieben, mit Antikörper mAK-199 oder mAK-1 beschichtet wurden. Der nachfolgende Ablauf ist wie unter Beispiel 4 b) beschrieben.
   Wie aus Abbildung 1 ersichtlich, stören keine Komponenten aus Humanserum bei der Bestimmung von TNF mittels monoklonaler Antikörper.
d) Kreuzreaktion der Antikörper mit Lymphotoxin
   Der Nachweis einer möglichen Kreuzreaktion der Antikörper mit Lymphotoxin wurde entsprechend Beispiel le getestet, wobei die Näpfchen der Mikrotiterplatte mit aufgereinigtem rekomoinantem Lymphotoxin beschichtet wurden. Es wurde keine Bindung der Antikörper mAK-1, mAK-114, mAK-195 und mAK-199 an Lymphotoxin gefunden.

### Beispiel 5

### Nachweis von TNF im Serum durch Western-Blotting

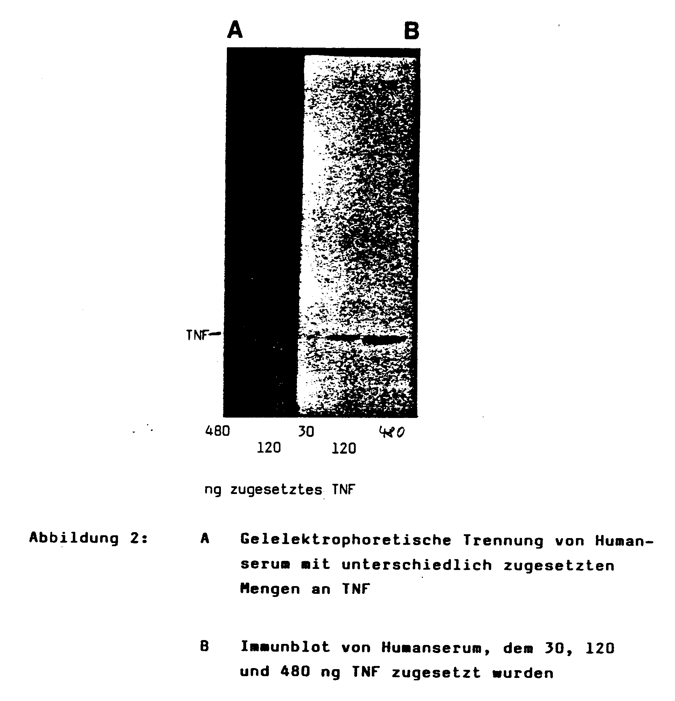

Humanserum mit unterschiedlich zugesetzten Mengen an TNF wurde im 12,5 % Gel nach Laemmli (J. Mol. Biol. 1973, Vol. 80, 575 - 599) gelelektrophoretisch aufgetrennt. Die Methode des Western Blotting wurde, wie bei Burnett, W. N. (Anal. Biochem. 1981, Vol. 112, 195 - 203) und Reines, D. et al. (J. Biol. Chem. 1985, Vol. 260, 1133 - 1139) beschrieben, durchgeführt. Die Proteine des Gels wurden über Nacht auf Nitrozellulosemembran (Schleicher und Schüll) geblottet. Die Nitrozellulosemembran wurde für drei Stunden bei Raumtemperatur mit 1 %iger Gelatinelösung (Bio-Rad, zu 1 l PBS wurden 10 g Gelatine gegeben) inkubiert. Anschließend wurde die Nitrozellulosemembran mit 20 ml Antikörperlösung, die auf 1 µg/ml in Puffer (0,1 % Gelatine in PBS) verdünnt wurde, für zwei Stunden bei Raumtemperatur inkubiert. Der Überstand wurde dekantiert und die Membran mehrmals gewaschen. Unter Verwendung von Peroxidase-markiertem Anit-Maus Immunoglobulin wurde TNF auf der Nitrozellulosemembran sichtbar gemacht. Die Nachweisgrenze liegt bei 30 ng TNF. Wie aus Abbildung 2 ersichtlich ist, reagiert der monoklonale Antikörper mAK-195 mit keiner Komponente aus humanem Serum und kann somit als spezifisch für TNF angesehen werden.

### Beispiel 6 Neutralisation von TNF

Der schützende Effekt des mAK-195 gegen TNF wurde unter in-vivo-Bedingungen in männlichen Balb/c - (Versuch 1, 2, 4) und C3H/HeN - (Versuch 3) Mäusen untersucht. 4-6 Wochen alte Mäuse wurden randomisiert und in Gruppen von 3 bzw. 5 Tieren aufgeteilt. Die Substanzen wurden intravenös in die laterale Schwanzvene gegeben (Applikationsvolumen 10 ml/kg). TNF wurde vor der Injektion in Puffer A (150 mM NaCl und 0,18 % Rinderserumalbumin (Sigma, RIA-grade) gelöst und bei 4-10°C für 6 Stunden gelagert. Nach dieser Zeit zeigte TNF die größte Toxizität. TNF wurde zuerst gegeben, der mAK195 15-30 Minuten danach. Die Tötungsraten wurden nach 24 Stunden bestimmt. Tabelle 5 zeigt die erhaltenen Ergebnisse (3/5 bedeutet, daß 3 von 5 Tieren gestorben waren).

**Tabelle 5**

| Applizierte Substanzen | Versuch-Nr. | | | |
|---|---|---|---|---|
| | 1 | 2 | 3 | 4 |
| Puffer A | 0/3 | 0/5 | 0/5 | 0/5 |
| mAK 10 mg/kg | 0/3 | 0/5 | 0/5 | 0/5 |
| mAK 5 mg/kg | 0/3 | 0/5 | 0/5 | 0/5 |
| TNF 1 mg/kg | 3/3 | 0/5 | 4/5 | 4/5 |
| TNF 1 mg/kg + mAK 1 mg/kg | 1/3 | 2/5 | 1/5 | 3/5 |
| TNF 1 mg/kg + mAK 5 mg/kg | 3/3 | 0/5 | 0/5 | 0/5 |
| TNF 2 mg/kg | 3/3 | 5/5 | 1/5 | 4/5 |
| TNF 2 mg/kg + mAK 2 mg/kg | 3/3 | 4/5 | 2/5 | 5/5 |
| TNF 2 mg/kg + mAK 10mg/kg | 0/3 | 0/5 | 0/5 | 0/5 |

Wie aus Tabelle 5 zu entnehmen ist, kann der neutralisierende Antikörper mAK195 eine tödliche Dosis von TNF in der Maus neutralisieren. Die Überlebensraten der Tiere sind abhängig vom Gewichtsverhältnis Antikörper zu TNF. Eine vollständige Aufhebung der TNF-Toxizität ist bei einem Verhältnis von 5 : 1 zu beobachten. Unter der Annahmne, daß TNF als Trimeres vorliegt (FEBS Lett. 211, 179 (1987)), entspricht dies einem molaren Verhältnis Antikörper zu TNF von 1,6 zu 1.

TNF läßt sich nicht durch nicht neutralisierende Antikörper in der Maus neutralisieren.

## Patentansprüche

1. mAK 195 erhältlich aus der Zellinie ECACC 87 050801.

2. Hybridzellinie ECACC 87 050801.

3. Verfahren zur Herstellung des monoklonalen Antikörpers mAK 195 gemäß Anspruch 1, dadurch gekennzeichnet, daß man die Zellinie ECACC 87 050801 in vitro oder in vivo kultiviert und den Antikörper durch Aufreinigung von Überständen der in vitro Zellkultur oder der Ascitesflüssigkeit isoliert.

4. Verwendung des monoklonalen Antikörpers mAK 195,
a) zum Nachweis von natürlichem und rekombinantem TNF,
b) zur Bestimmung von TNF in biologischen Flüssigkeiten,
c) zur Herstellung von Immuntests für die Bestimmung von TNF,
d) zur Isolierung von TNF mittels Immunadsorptionschromatographie.

5. mAK 195 zur Verwendung bei der Bekämpfung von Krankheiten.

## Claims

1. mAK 195 obtainable from the cell line ECACC 87 050801.

2. The hybrid cell line ECACC 87 050801.

3. A process for preparing the monoclonal antibody mAb 195 as claimed in claim 1, which comprises cultivating the cell line ECACC 87 050801 in vitro or in vivo, and isolating the antibody by purification of supernatants of the in vitro cell culture or of the ascitic fluid.

4. The use of the monoclonal antibody mAb 195
a) to detect natural and recombinant TNF,
b) to determine TNF in biological fluids,
c) to prepare immunoassays for the determination of TNF,
d) to isolate TNF using immunoadsorption chromatography.

5. mAK 195 for use in controlling diseases.

## Revendications

1. mAK 195 pouvant être obtenu à partir de la lignée cellulaire ECACC 87 050801.

2. Lignée cellulaire hybride ECACC 87 050801.

3. Procédé de préparation de l'anticorps monoclonal mAK 195 selon la revendication 1, caractérisé par le fait que l'on cultive la lignée cellulaire ECACC 87 050801 in vitro ou in vivo et l'on isole l'anticorps par purification du surnageant de la culture cellulaire in vitro ou du liquide ascitique.

4. Utilisation de l'anticorps monoclonal mAK 195
a) pour déceler TNF naturel et recombinant,
b) pour déterminer TNF dans des liquides biologiques,
c) pour préparer des immunotests pour la détermination de TNF,
d) pour isoler TNF par chromatographie d'immunoabsorption.

5. mAK 195 pour l'utilisation dans la lutte contre des maladies.
